# FASCICULE DE BREVET EUROPEEN

(11) **EP 0 503 991 B1**
(45) Date de publication et mention de la délivrance du brevet: **23.09.1998**
(21) Numéro de dépôt: 92400505.1
(22) Date de dépôt: 27.02.1992
(51) Int. Cl.: C07K 1/00, C07K 14/00, A61K 38/00

(54) **Procédé de préparation à l'échelle industrielle d'un concentré de facteur von Willebrand humain standardisé, de très haute pureté, approprié à un usage thérapeutique**
Verfahren zur Herstellung in industriellem Massstab eines Konzentrates von hochgereinigtem, standardisiertem humanen von Willebrand Faktor, das für therapeutische Anwendung geeignet ist
Process for preparing a highly purified standardized concentrate of human von Willebrand factor on industrial scale, appropriate for therapeutic use

(30) Priorité: 08.03.1991 FR 9102804
(43) Date de publication de la demande: 16.09.1992
(73) Titulaire: CENTRE REGIONAL DE TRANSFUSION SANGUINE DE LILLE, 59012 Lille (FR)
(72) Inventeur: Burnouf-Radosevich, Miryana, F-59136 Wavrin (FR); Burnouf, Thierry, F-59136 Wavrin (FR)
(74) Mandataire: Lepeudry-Gautherat, Thérèse

(56) Documents cités:
- EP-A- 359 593
- BIOCHEMISTRY. vol. 25, no. 11, 3 Juin 1986, EASTON, PA US pages 3146 - 3155; M. W. CHOPEK ET AL.: 'HUMAN VON WILLEBRAND FACTOR: A MULTIVALENT PROTEIN COMPOSED OF IDENTICAL SUBUNITS.'
- VOX SANGUINIS vol. 60, no. 1, Janvier 1991, BASEL, CH pages 8 - 15; T. BURNOUF ET AL.: 'A HIGHLY PURIFIED FACTOR VIII:c CONCENTRATE PREPARED FROM CRYOPRECIPITATE BY ION-EXCHANGE CHROMATOGRAPHY.'
- CHEMICAL ABSTRACTS, vol. 96, no. 19, 10 Mai 1982, Columbus, Ohio, US; abstract no. 158527, T. BURNOUF ET AL.: 'COMBINATION OF GELATIN-CRYODESSICATED AGAROSE FOR THE SELECTIVE EXTRACTION OF HUMAN PLASMA FIBRONECTIN.' page 379 ; &REV. FR. TRANSFUS. IMMUNO-HEMATOL. vol. 24, no. 6, 1981, pages 559 - 570

## Description

L'invention concerne un procédé de préparation à l'échelle industrielle d'un concentré de facteur von Willebrand humain, standardisé, de grande pureté, à très haute activité spécifique et présentant un taux élevé de multimères de haut poids moléculaire, destiné notamment à un usage thérapeutique.

Le facteur von Willebrand (fvW) est la plus grande molécule connue en circulation dans le plasma. Il est constitué d'un ensemble àe multimères liés par des ponts S-S, dont l'élément de base a un poids moléculaire voisin de 260 kilodaltons (KDa). La plus petite forme de fvW, dans le plasma, est un dimère de 440-500 KDa et les formes les plus grandes sont des multimères de ce dimère dont le poids moléculaire peut atteindre 20 millions de daltons. Cet assemblage des sous-unités en multimères peut être spécifique des cellules dans lesquelles il est opéré, le fvW étant synthétisé et polymérisé dans les mégacaryocytes et dans les cellules endothéliales.

Ce facteur joue un rôle essentiel dans l'hémostase par deux fonctions distinctes : comme protéine d'adhésion il permet la dispersion, l'adhérence et l'aggrégation des plaquettes sanguines sur le sous-endothelium vasculaire (et participe ainsi au processus de cicatrisation rapide des vaisseaux lésés) et, d'autre part, il assure la stabilisation et le transport du Facteur VIII dans la circulation sanguine.

Une déficience congénitale en fvW ou une anomalie structurale de ce facteur entraine la maladie de Willebrand qui se manifeste par des hémorragies surtout cutanées et des muqueuses. Cette maladie est très hétérogène dans son expression clinique et pose de graves problèmes en cas d'intervention chirurgicale. Le traitement de la maladie de Willebrand s'impose pour corriger les anomalies de l'hémostase primaire (temps de saignement) et de la coagulation (temps de céphaline activée et activité coagulante du Facteur VIII).

La maladie se traite par thérapie de substitution par des dérivés de plasma humain enrichis en fvW (par exemple la fraction cryoprécipitée du plasma ou des concentrés de Facteur VIII contenant suffisamment de fvW associé à celui-ci). Cependant ces produits ne sont pas standardisés en vue du traitement de la maladie de Willebrand. De plus les fractions peu purifiées de plasma sanguin, en particulier le cryoprécipité, ne sont pas exemptes de risques de contamination virale parce qu'elles ne sont souvent pas soumises à un traitement efficace d'inactivation virale. En outre, elles entraînent une surcharge en protéines contaminantes dont le patient n'a pas besoin et qui peuvent entraîner des réactions immunologiques après des injections multiples.

Le Facteur VIII purifié, au contraire, peut être soumis à un traitement d'inactivation virale efficace mais son procédé de purification a été optimisé en vue du traitement de l'hémophilie A et non en vue du traitement de la déficience en fvW.

Ainsi les procédés de préparation du Facteur VIII récemment mis au point, comme l'immunoaffinité et la purification par échange d'ions, sont de plus en plus performants et produisent des concentrés qui ne contiennent plus suffisamment de fvW pour être efficaces dans le traitement de la maladie de Willebrand.

C'est pour combler ce besoin d'un traitement efficace pour la maladie de Willebrand que la Demanderesse a mis au point un nouveau procédé de purification du fvW à l'échelle industrielle, qui permet de rentabiliser au mieux l'extraction de diverses molécules plasmatiques. En particulier il permet, dans une première étape, la préparation d'un concentré de Facteur VIII (selon un procédé décrit dans la demande de brevet européen 0 359 593) et, ultérieurement, la récupération d'une fraction enrichie en facteur von Willebrand, à partir du même lot de cryoprécipité, ce qui permet de rentabiliser au mieux l'utilisation du plasma humain. La fraction de fvW ainsi obtenue est ensuite purifiée par deux étapes de chromatographie supplémentaires ce qui aboutit à un concentré de fvW de très haute pureté.

La complexité de la molécule du facteur von Willebrand rend sa préparation très difficile. Des méthodes de purification à petite échelle, c'est-à-dire de 5 à 2000 ml, dans des buts d'étude analytique ont déjà été décrites mais ces méthodes n'ont pas pu être adaptées à l'échelle industrielle (Thorell et al. Thromb Res. 35, 1984, 431-450). De plus, le principe de rentabilisation maximale du cryoprécipité pour produire à la fois du FVIII et du fvW n'avait pas encore été envisagé.

Le facteur von Willebrand a été purifié par solubilité différentielle sur des composés sulfatés, en présence de glycine (Berntorp etal. Vox Sang. 56, 1989, 212-217 ; Winkelman et al. Vox Sang. 57, 1989, 97-103) et par différentes méthodes de séparations chromatographiques, par tamisage moléculaire (Perret et al. Haemostasis 14, 1984, 289-295) ou par échange d'ions (Austen et al. Thromb Haemostas.48, 1982, 46-48 ; Harrison et al. Thromb. Res. 50, 1988, 295-304), mais ces techniques montrent d'assez faibles rendements en facteur von Willebrand ou une faible capacité du gel ou ne permettent pas la récupération simultanée du FVIII et du fvW, ce qui les rendent moins intéressantes pour une application industrielle.

Austen et al. (1982, déjà cité) obtiennent un concentré de faible pureté (8 U Ag/mg protéine) et avec un rendement assez faible, probablement dû à leurs conditions de chromatographie très drastiques (pH 5,5).

Harrisson et al. (1988, déjà cité) utilisent comme matrice du dextran-sulfate-sépharose ; ce matériau n'a qu'une faible capacité de rétention du fvW et le fvW ainsi produit n'a qu'une activité spécifique de 2-4 U/mg protéine.

De plus la plupart de ces produits contiennent une proportion importante de formes dénaturées ou inactives, à côté des formes multimériques actives,comme l'indique le rapport activité cofacteur de ristocétine (RCo)/antigène qui est de l'ordre de 0,08 à 0,8 (Lawrie et al. Br. J. Haematol. 1989, 73,100), ce qui les rend moins efficaces pour un usage thérapeutique dans la maladie de Willebrand. Au contraire, le procédé de la Demanderesse permet d'obtenir un concentré de fvW dont le rapport RCo/Ag est supérieur à un, ce qui est comparable à celui du fvW natif, dans le pool de plasma.

La présente invention concerne un procédé de préparation de concentré de facteur von Willebrand à usage thérapeutique, ledit procédé permettant de préparer des lots standardisés dérivant chacun de très grands volumes de plasma (4000 litres ou plus), à partir desquels on a déjà récupéré du concentré de FVIII, ce qui rentabilise au mieux l'utilisation du cryoprécipité.

Plus particulièrement, la présente invention concerne un procédé de préparation d'un concentré de facteur von Willebrand qui comprend la combinaison de trois étapes successives de chromatographie permettant un enrichissement en multimères de haut poids moléculaire, associés à l'activité biologique du fvW. Le matériau de départ est la fraction cryoprécipitée du plasma humain, soumise à une étape classique de prépurification par adsorption sur hydroxyde d'aluminium. Ce matériau est ensuite soumis à une inactivation virale, par exemple par un traitement par solvant-détergent, avant sa purification.

Le procédé de purification selon la présente invention comprend une combinaison de trois étapes successives de chromatographie d'un sous-produit de la production du FVIII, les deux premières étant des chromatographies d'échanges d'ions et la troisième étant une chromatographie d'affinité.

Les deux chromatographies d'échanges d'ions sont effectuées sur la même résine de polymère vinylique greffé de groupements DEAE (diethylaminoethyl), plus particulièrement sur des colonnes de DEAE-Fractogel^{(M)} TSK 650 (Merck), équilibrées avec du tampon comprenant du citrate trisodique 0,01 M, du chlorure de sodium 0,11 M, du chlorure de calcium 0,001 M, de la glycine 0,12 M et de la lysine 0,016 M, à pH7.

Le DEAE-Fractogel^{(M)} TSK 650 est un gel synthétique hydrophile. Le support est un copolymère d'oligoethylèneglycol, de glycidineméthacrylate et de pentaérythritol-diméthacrylate sur lequel sont greffés des groupements diéthylaminoéthyl comme -O-CH₂-CH₂N⁺(C₂H₅)₂HC1, ce qui constitue un échangeur d'anions faiblement alcalin. Le DEAE-Fractogel TSK 650 est disponible sous deux tailles de particules (après réhydratation) : le type S (0,025-0,050 mm) et le type M (0,045 - 0,090 mm) ; les deux types peuvent être utilisés pour la présente invention.

La fraction cryoprécipitée du plasma, prépurifiée et ayant subi un traitement d'inactivation virale, est appliquée sur la première colonne de chromatographie qui retient une grande partie du facteur von Willebrand. Celui-ci est ensuite élué par une augmentation de la concentration de chlorure de sodium du tampon à 0,14 -0,15 M.

La fraction ainsi éluée, enrichie en facteur von Willebrand, est appliquée sur la deuxième colonne de chromatographie, dans les mêmes conditions que sur la première. La fraction injectée sur cette deuxième colonne étant déjà débarrassée de beaucoup de protéines (surtout le FVIII et la fibronectine), qui entraient en compétition pour les sites d'adsorption, la capacité d'adsorption du fvW sur la deuxième colonne est beaucoup plus grande. Après élimination du filtrat et rinçage de la colonne avec le tampon d'équilibrage, le fvW adsorbé est élué par une augmentation de la concentration de chlorure de sodium du tampon à 0,15 - 0,17 M.

Grâce à la grande capacité d'adsorption et à l'efficacité de la résine de DEAE-Fractogel vis-à-vis du fvW, celui-ci est récupéré avec une haute activité spécifique ( > 150 U RCo/ml) ce qui permet d'éviter une étape supplémentaire de concentration par ultrafiltration.

La fraction ainsi éluée est soumise à une chromatographie d'affinité sur colonne de gélatine-sépharose, dans le même tampon d'équilibrage, ce qui permet d'éviter une étape de dialyse ou d'ultrafiltration pour modifier la concentration saline ; cette colonne retient les molécules de fibronectine résiduelle qui contaminaient encore le fvW. Le choix du gel associé à la gélatine n'est pas critique : on peut également utiliser de la gélatine-Ultrogel^{(M)}, de la gélatine-Spherodex^{(M)} ou de la gélatine-Fractogel^{(M)}. On utilise de préférence de la gélatine-sépharose^{(M)}, qui fixe jusqu'à 5-10 mg de fibronectine/ml dans les conditions utilisées dans le procédé selon l'invention.

Le facteur von Willebrand purifié dans ces conditions ne se lie pas au gel et passe dans le filtrat ; comme l'étape de chromatographie en présence de gélatine n'implique pas de dilution importante du fvW, il n'est pas nécessaire de le concentrer (par exemple par ultrafiltration) et il peut être directement conditionné et lyophylisé. L'absence d'enzymes protéolytiques dans le produit final assure sa stabilité au cours de la filtration stérilisante et de la lyophilisation ce qui évite l'addition d'agents stabilisants.

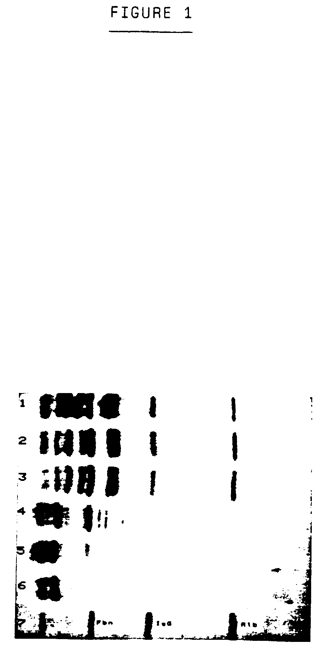

Le concentré de facteur von Willebrand obtenu par le procédé selon la présente invention présente un taux de purification exceptionnement élevé de > 10 000 fois par rapport au plasma de départ et son activité spécifique est de 345 U CBA/mg de protéines (unités de mesure de l'activité de liaison du collagène) et de > 100 U RCo/mg de protéines (unités d'activité de cofacteur de ristocétine). Le rôle de chaque étape de chromatographie dans la purification du fvW est illustré par la figure 1.

L'amélioration de la qualité du produit au cours des étapes successives de purification a été surveillée tout particulièrement en fonction de la proportion des molécules à l'état de multimères de haut poids moléculaire (c'est-à-dire des formes moléculaires à haute activité biologique) par analyse électrophorétique.

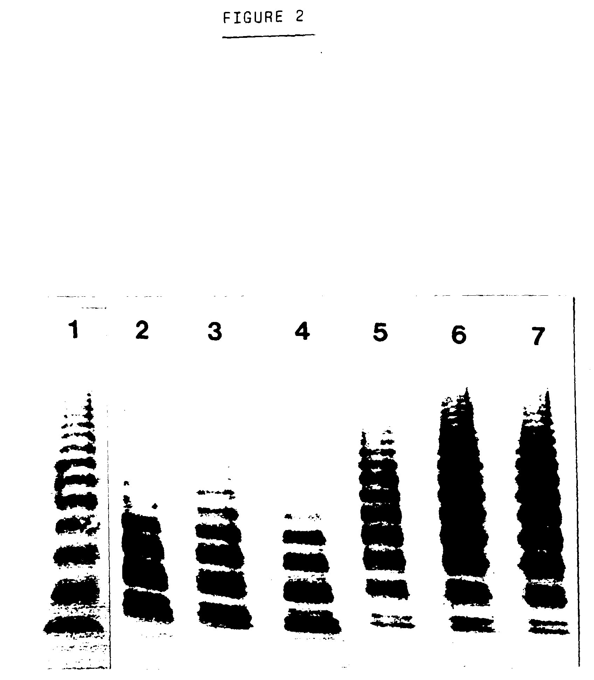

Cette analyse révèle un enrichissement progressif en multimères > 4 (Figure 2) qui représentent 79% du fvW dans la préparation finale, par rapport à 70% dans le plasma natif et alors que la cryoprécipitation en fait perdre la moitié. De manière inattendue, c'est la chromatographie sur DEAE-Fractogel^{(M)} qui favorise cette rétention sélective des multimères de très grande taille et élimine dans le filtrat les formes de petite taille, de structure anormale (ayant subi une protéolyse partielle) et de faible activité.

Le concentré de fvW standardisé de grande pureté à haute activité spécifique et à haute teneur en multimères de haut poids moléculaire, obtenu par le procédé selon la présente invention est donc particulièrement bien adapté à un usage thérapeutique dans les différentes formes de la maladie de Willebrand, ce qui est confirmé par des études cliniques préliminaires. Celles-ci montrent, en particulier, que le concentré àe fvW raccourcit efficacement le temps de saignement au cours des hémorragies.

Des tests in vitro ont confirmé l'identité de ses propriétés biochimiques et physiologiques avec celles de la molécule native, en particulier, -sa capacité de fixer les plaquettes sanguines dans un dispositif de perfusion, et -sa capacité de se lier au Facteur VIII endogène.

La haute pureté du concentré de facteur von Willebrand obtenue par le procédé selon la présente invention permet également d'envisager son application dans divers usages de laboratoire (d'analyse fine de structure, d'étude de fonctionalité, de diagnostic...) et de production d'anticorps spécifiques.

Le concentré selon la présente invention peut également être utilisé comme stabilisant au cours de la production du Facteur VIII par des cellules transformées par génie génétique et au cours des étapes de purification du Facteur VIII ainsi produit.

L'exemple suivant illustre un mode de réalisation de la présente invention sans toutefois en limiter la portée.

### EXEMPLE

### - Matériau de départ.

Le cryoprécipité est préparé à partir de plasma frais récolté en présence de citrate de sodium (4%) ou de solution anticoagulante CPD (citrate, phosphate, dextrose), et congelé au plus tard 6 heures après son prélèvement. Le plasma est congelé à -60°C puis conservé à -35°C. Les lots de plasma contiennent de 1800 à 2000 litres et sont rassemblés par lots de 4000 litres pour chaque mise en ouvre du procédé. En vue de la décongélation, le plasma est placé dans une chambre à -7°C pendant au moins 12 heures pour assurer un réchauffement lent et régulier puis il est dégelé dans une enceinte thermostatée à 0 - 2°C, sous agitation constante. Le cryoprécipité (qui représente environ 9 g/litre de plasma) est récupéré par centrifugation à froid.

Après centrifugation, le cryoprécipité récupéré est remis en solution et adsorbé sur hydroxyde d'aluminium pour éliminer les principaux contaminants, c'est-à-dire les composants du complexe prothrombinique (particulièrement le Facteur VII), le Facteur XII. Le surnageant est ensuite refroidi à 15°C (ce qui élimine en partie le fibrinogène et la fibronectine).

Ce traitement permet une récupération de 80 à 86% du mélange Facteur VIII - facteur von Willebrand du cryoprécipité ; l'activité spécifique du Facteur VIII est de 0,7 UI/mg et celle du fvW de 0,6 U RCo/mg (activité cofacteur de ristocétine) et de 1,2 U CBA/mg (activité de liaison du collagène).

### - Traitement d'inactivation virale.

La solution contenant le Facteur VIII - facteur von Willebrand est soumise à un traitement par solvant-détergent connu pour son efficacité contre les virus à enveloppe lipidique (Horowitz et al., 1985, Trasfusion, 25, 516-522.) et qui comprend une incubation de 8 heures à 25°C en présence de 0,3 % tri-n-butyl phosphate (TnBP) et de 1% de Tween 80.

Après ce traitement on retrouve 95% de l'activité des Facteurs VIII et von Willebrand mesurée à l'étape précédente. L'électrophorèse permet de contrôler que le fvW est toujours sous forme multimérique.

### - Procédé de séparation chromatoqraphique.

La purification du facteur von Willebrand apparaît comme une dérivation du procédé de purification du Facteur VIII décrit par la Demanderesse dans la demande de brevet européen 0 359 593.

La première chromatographie est réalisée sur une colonne de DEAE-Fractogel^{(M)} TSK 650 (Merk). Le tampon d'équilibrage contient du citrate trisodique (0,01 M), du chlorure de calcium (0,001 M), du chlorure de sodium (0,11 M), de la glycine (0,12 M) et de la L-lysine (0,016 M). Le facteur von Willebrand, le Facteur VIII et la fibronectine sont retenus par la colonne ; les protéines contaminantes (principalement le fibrinogène et des IgG) faiblement ou pas fixées par la colonne et les résidus du traitement d'inactivation virale sont éliminés par plusieurs lavages successifs avec le même tampon.

La colonne est utilisée avec une vitesse de flux linéaire de 100 cm/heure.

Dans ces conditions d'utilisation, la colonne utilisée présente une capacité de rétention du fvW de environ 75% (mesuré sous forme d'antigène, Ag), le reste étant perdu dans le filtrat. Cette capacité de fixation correspond à 45 U d'Ag fvW/ml de gel.

Le facteur von Willebrand est désorbé de la colonne par une augmentation de la concentration en NaCl du tampon à 0,15M. La fraction de fvW récoltée contient 30 à 35 % du fvW initial mais 40 % de celui-ci reste co-adsorbé avec le Facteur VIII et sera co-élué avec celui-ci par une seconde augmentation de la concentration du tampon à 0,25 M et sera co-purifié avec celui-ci.

La fraction contenant le fvW élué de cette première colonne est réinjectée sur une seconde colonne, identique à la première et dans les mêmes conditions, après une légère dilution avec le tampon d'équilibrage, pour ajuster la force ionique de la fraction fvW à l'équivalent de 0,11 M en chlorure de sodium.

La fraction injectée étant déjà débarrassée de contaminants et du Facteur VIII qui entraient en compétition pour la capacité de fixation des sites d'adsorption de la première colonne, on observe pour la seconde, une capacité de fixation beaucoup plus grande, de 320 U d'Ag fvW/ml de gel.

Le fvW est désorbé par une augmentation de la concentration en NaCl du tampon à 0,17 M.

Cette deuxième chromatographie permet un facteur de concentration de 8 à 10 fois par rapport à la précédente ce qui évite des étapes supplémentaires de concentration par ultrafiltration, par exemple. Dans les différents systèmes de mesure habituels, l'éluat contient les taux ou activités de fvW suivants :
- antigène (Ag) 88 ± 9 U/ml
- cofacteur de ristocétine : 97 ± 19 U/ml
- capacité de fixation au collagène (CBA) : 149 ± 13 U/ml.
- multimères (≥ 4) de haut poids moléculaire 79%

Le rapport CBA/Ag de 1,69 montre que l'activité du fvW est bien préservée. Ceci est en accord avec le haut pourcentage (79%) de multimères de haut poids moléculaire.

L'analyse électrophorétique de cet éluat du fvW révèle la présence d'une légère contamination par de la fibronectine et de l'inter-alpha trypsine inhibiteur (ITI).

Ce second éluat de fvW est soumis à une troisième étape de purification sur colonne de gélatine-Sepharose CL4B (Pharmacia) équilibrée avec le tampon d'élution de la colonne précédente, pour éliminer la fibronectine.

Ce gel de chromatographie d'affinité a une capacité de rétention de la fibronectine de > 5 mg/ml ce qui permet de réduire ce contaminant à des quantités indétectables (< 4 mg/l) dans la fraction fvW.

Le fvW purifié se retrouve dans le filtrat de cette dernière étape et peut être directement conditionné et lyophilisé.
- l'analyse électrophorétique ne détecte plus aucun contaminant,
- le contenu en fvW est de 205 U Ag/mg protéine,
- son activité spécifique est de 345 U CBA/mg de protéine
   et 186-220 U RCo/mg de protéine.
Le degré total de purification par rapport au plasma initial est de > 10 000 fois.

Le fvW obtenu en fin de purification est constitué à 65 à 80 % de multimères de haut poids moléculaire, c'est-à-dire un taux comparable à celui du plasma de départ qui est de 70 %, comme le montre l'analyse électrophorétique sur agarose-SDS.

La stabilité du concentré a été étudiée à l'état liquide à température ordinaire pendant 24 heures : on ne détecte aucune trace de protéolyse ni de changement d'activité spécifique.

L'absence totale d'activité thrombogène du concentré a été contrôlée par les tests classiques de NAPTT ("non activated partial thromboplastin time"). L'absence totale de thrombine, de PKA et de kallikréine a été demontrée.

Le concentré final obtenu ne nécessite aucune addition de stabilisant.
Figure 1 : SDS-PAGE des fractions de fvW au cours de la purification. bande 1 : cryoprécipité ; bande 2 : cryoprécipité traité au solvant-détergent ; bande 3 : fraction non liée au DEAE-Fractogel ; bande 4 : ler éluat de fvW ; bande 5 : 2e éluat de fvW ; bande 6 : fraction non liée au gel à la gélatine ; bande 7 : standards.
   Fbn = fibronectine
   IgG = immunoglobulines
   Alb = albumine
Figure 2 : Distribution des multimères du fvW dans les fractions en cours de purification, après électrophorèse sur agarose-SDS.
   bande 1 : plasma natif ; bande 2 : cryoprécipité ; bande 3 : cryoprécipité traité au solvant-détergent ; bande 4 : fraction non liée au DEAE-Fractogel ; bande 5 : ler éluat de fvW ; bande 6 : 2e éluat de fvW ; bande 7 : fraction non liée au gel à la gélatine.

## Revendications

1. Procédé de préparation d'un concentré du facteur von Willebrand humain standardisé, à partir d'une fraction cryoprécipitée de plasma, prépurifiée sur hydroxyde d'aluminium, caractérisé
- en ce qu'il comprend une combinaison de trois étapes successives de séparation chromatographique, les deux premières étant des chromatographies d'échange d'ions sur une résine de type polymère vinylique à larges pores portant des groupements DEAE et la troisième étant une chromatographie d'affinité sur gélatine couplée à un gel,
- et que les trois chromatographies sont réalisées dans un tampon comprenant du citrate trisodique, du chlorure de sodium, du chlorure de calcium, de la glycine et de la lysine.

2. Procédé selon la revendication 1, caractérisé en ce que les deux chromatographies d'échange d'ions sont réalisées sur colonnes de DEAE-Fractogel^{®}.

3. Procédé selon la revendication 1 ou 2, caractérisé en ce que la chromatographie d'affinité est réalisée sur colonne de gélatine-Sepharose^{®}.

4. Procédé selon l'une quelconque des revendications 1 à 3, caractérisé en ce que le tampon des chromatographies comprend du citrate trisodique 0,01 M, du chlorure de calcium 0,001 M, de la glycine 0,12 M et de la lysine 0,016 M et du chlorure de sodium en concentration variant de 0,11 M à 0,17 M.

5. Procédé selon la revendication 1 ou 2, caractérisé en ce que le facteur von Willebrand qui s'est adsorbé sur la première colonne de chromatographie est élué par une augmentation de la concentration de chlorure de sodium du tampon à 0,14-0,15 M, que le facteur von Willebrand qui s'est adsorbé sur la deuxième colonne de chromatographie est élué par une augmentation de la concentration de chlorure de sodium du tampon à 0,15-0,17 M, et qu'il est ensuite débarrassé de la fibronectine résiduelle par adsorption par affinité sélective sur la troisième colonne de chromatographie sur gélatine.

6. Procédé selon l'une quelconque des revendications 1 à 5, caractérisé en ce que le facteur von Willebrand concentré est présent dans le filtrat de la colonne de gélatine et est récolté sans autre traitement, conditionné et lyophilisé.

## Patentansprüche

1. Verfahren zur Herstellung eines standardisierten Konzentrats des humanen Von-Willebrand-Faktors aus einer bei tiefer Temperatur gefällten, auf Aluminiumhydroxid vorgereinigten Plasmafraktion, dadurch gekennzeichnet,
- daß es eine Kombination von drei aufeinanderfolgenden Schritten der chromatographischen Trennung umfaßt, wobei die beiden ersten Schritte Ionenaustauschchromatographien auf einem Harz vom Typ eines DEAE-Gruppierungen tragenden, großporigen Vinylpolymers sind und der dritte Schritt eine Affinitätschromatographie auf an ein Gel gekoppelter Gelatine ist,
- und daß die drei Chromatographien in einem Puffer ausgeführt werden, der Trinatriumcitrat, Natriumchlorid, Calciumchlorid, Glycin und Lysin umfaßt.

2. Verfahren nach Anspruch 1, dadurch gekennzeichnet, daß die beiden Ionenaustauschchromatographien auf Kolonnen von DEAE-Fractogel^{®} ausgeführt werden.

3. Verfahren nach Anspruch 1 oder 2, dadurch gekennzeichnet, daß die Affinitätschromatographie auf einer Kolonne von Gelatine-Sepharose^{®} ausgeführt wird.

4. Verfahren nach einem der Ansprüche 1 bis 3, dadurch gekennzeichnet, daß der Puffer für die Chromatographien 0,01 mol/l Trinatriumcitrat, 0,001 mol/l Calciumchlorid, 0,12 mol/l Glycin und 0,016 mol/l Lysin sowie Natriumchlorid in einer Konzentration enthält, die von 0,11 mol/l bis 0,17 mol/l variiert.

5. Verfahren nach Anspruch 1 oder 2, dadurch gekennzeichnet, daß der Von-Willebrand-Faktor, der auf der ersten chromatographischen Kolonne adsorbiert ist, durch Erhöhung der Natriumchlorid-Konzentration des Puffers auf 0,14 - 0,15 mol/l eluiert wird, daß der Von-Willebrand-Faktor, der auf der zweiten chromatographischen Kolonne adsorbiert ist, durch eine Erhöhung der Natriumchlorid-Konzentration des Puffers auf 0,15-0,17 mol/l eluiert wird und daß er anschließend durch Adsorption infolge selektiver Affinität auf der dritten chromatographischen Kolonne auf Gelatine vom restlichen Fibronectin befreit wird.

6. Verfahren nach einem der Ansprüche 1 bis 5, dadurch gekennzeichnet, daß der konzentrierte Von-Willebrand-Faktor im Filtrat der Gelatine-Kolonne vorhanden ist und ohne weitere Behandlung gesammelt, konditioniert und gefriergetrocknet wird.

## Claims

1. A process for preparing a standardized concentrate of von Willebrand factor, starting from a cryoprecipitated plasma fraction, prepurified on aluminium hydroxide, characterized
- in that it includes a combination of three successive chromatographic separation steps, the first two being ion exchange chromatography on a vinyl polymer type resin with large pores and grafted with DEAE groups and the third being an affinity chromatography on gelatin-grafted gel,
- and that said three chromatographies are performed in a buffer including trisodium citrate, sodium chloride, calcium chloride, glycine and lysine.

2. The process according to claim 1, characterized in that the two ion exchange chromatographies are performed on DEAE-Fractogel^{®} columns.

3. The process according to claim 1 or 2, characterized in that the affinity chromatography is performed on a gelatin-Sepharose^{®} column.

4. The process according to any of claims 1 to 3, characterized in that the chromatographic buffer contains 0.01 M trisodium citrate, 0.001 M calcium chloride, 0.12 M glycine and 0.016 M lysine and a concentration of sodium chloride which varies from 0.11 to 0.17 M.

5. The process according to claim 1 or 2, characterized in that the von Willebrand factor which is adsorbed on the first chromatography column is eluted by increasing the buffer sodium chloride concentration to 0.14-0.15 M, and that the von Willebrand factor which is adsorbed on the second chromatography column is eluted by increasing the buffer sodium chloride concentration to 0.15-0.17 M and that it is further cleared of residual fibronectin by selective affinity adsorption on the third gelatin chromatography column.

6. The process according to any of claims 1 to 5, characterized in that the von Willebrand factor concentrate is present in the filtrate of the gelatin column and that it is collected without any other treatment, dispensed and freeze-dried.
